Europäisches Patentamt

European Patent Office

Office européen des brevets

(11) Publication number: **0 496 142 A1**

## EUROPEAN PATENT APPLICATION

(21) Application number: **91300555.9**

(22) Date of filing: **24.01.91**

(51) Int. Cl.⁵: **A61B 1/04**, A61B 19/08, H04N 5/225

(43) Date of publication of application:
**29.07.92 Bulletin 92/31**

(84) Designated Contracting States:
**AT BE CH DE DK ES FR GB IT LI NL SE**

(71) Applicant: **SMITH & NEPHEW DYONICS INC**
**160 Dascomb Road**
**Andover, Massachusetts 01810(US)**

(72) Inventor: **Henley, Gary D.**
**13616 W. Hefner Road**
**Yukon, Oklahoma 73099(US)**
Inventor: **Milner, Larry Bruce**
**2717 N.W. 159th Street, Edmond**
**Oklahoma City, Oklahoma 73013(US)**
Inventor: **Dowdy, Clifford A.**
**14000 N. Frisco Place**
**Piedmont, Oklahoma 73078(US)**

(74) Representative: **Cole, William Gwyn, Dr.**
**Smith & Nephew p l c Corporate Patents and**
**Trade Marks Dept. Gilston Park**
**Harlow Essex CM20 2RO(GB)**

(54) **Video systems for surgical procedure.**

(57) A disposable video camera unit 10 for use in surgical procedures comprises a sterile cable connector 18 and sterile camera head 14. The camera head 14 and cable 16 is adapted for initial sterility and sterile packaging for subsequent singular use and is disposable after surgery. The camera head 14 may consist of a charge-coupled device type of image sensor 42 assembled in a compact camera head 82 of a type that can be completely sterilised sealed and packaged for prolonged shelf storage and eventual disposal after use.

FIG. 1.

The invention relates to endoscopic surgical methods and apparatus and, more particularly, to the use of disposable video imaging devices to increase sterilisation effectiveness while reducing overall equipment costs.

Numerous types of endoscopic viewing device have been used down through the years for observation of internal passages and organs of the body. Thus, flexible, scopic instruments of differing diameters and lengths serve various purposes as proctoscopes, cystoscopes and the like have sizes and lengths which are generally dictated by intended usage. More recently, developments in solid state imaging devices and other television pickup methods and advances in fibre optics light transmission have led to a number of new techniques wherein a selected form of imaging device is used in combination with a selected endoscope to enable monitored exploration of an internal part or wound with attendant probing and/or corrective instrument functioning in response to the visual picture as derived and reproduced on a suitable monitor. What is now generally referred to as arthoscopic surgery has come into being and presently enjoys widespread success using the various scope instruments with image sensing devices to direct surgical instruments.

The present invention is concerned with improvements in component devices used for endoscopic surgery. As usually required in such surgery, there is a camera control unit, recorders, monitor units and a suitable light source as used in conjunction with the scope instrument. In the present invention, it is proposed to provide a prepackaged, sterilised camera/connector unit in the form of a connector plug, a suitable length of connector cable and fluid-tight camera housing carrying a solid state image sensor. For a single surgical procedure, the pre-packaged assembly can be opened and plugged into the camera control unit, and the arthroscopic or other scope instrument to be utilised is then attached to the camera head. The equipment is then ready for a surgical procedure which, upon completion, will enable the camera/connector unit to be discarded.

Therefore, it is an object of the present invention to provide sterile arthroscopic surgery imaging device that exhibits greater readiness for use and reliablility of sterility.

It is also an object of the present invention to enable overall cost reduction in arthroscopic surgery practice.

It is yet another object of the invention to provide a disposable surgical imaging device that need not to be subjected to more than one initial potentially dangerous and damaging sterilisation procedure.

It is still further an object of the present invention to provide a method for providing sterile imaging devices that is safer and more reliable than those hitherto used.

Finally, it is an object of the present invention to provide a solid state imaging device that is compact and small in size while offering an absolute sterile condition through a surgical procedure with subsequent disposal.

According to the present invention there is provided a method enabling performance of arthroscopic surgery which includes the steps comprising:

providing from shelf storage a sterilised, disposable optical imager consisting of plug connector, connector cable and camera had with imaging electronics, said optical imager being secured in sterile pack in retention for single surgical procedure.

The image sensoring means, the associated electronic circuitry and optical systems for projecting a received optical image onto the sensory means to with the housing means for containing, sensing and optical and electronic systems are referred to herein as the camera head.

The housing may comprise a hollow body, preferably of a generally cylindrical construction. The housing may comprise two mating halfes which when juxtaposed will mate in a sealing engagement.

Each half may be provided with faces which will mate with a corresponding face on the other half, each face being provided with sealing means which, when the halves are mated, will form an air-impermeable, fluid impermeable seal, and thus prevent the ingress of fluids into the camera head body.

In a preferred embodiment of the invention the sealing means are provided by ridges formed extending around the respect mating faces. The ridge in one of the half housing is formed of a resilient material and may be located and retained in the housing in a conventional manner, for example in the manner used to retain O-rings. The other half housing may be provided with a ridge formed from a non-resilient material extending around the face. Upon mating of the two halves, the non-resilient ridge engages and deforms the ridge of resilient material, the abutment of the ridge surfaces forming the seal.

In an alternative or additional embodiment, the two housing shells may be located and retained by a system of dowels. Thus in the facing edge of one of the housing shells there are formed a plurality of outwardly extending pegs or dowels. An equal number of recesses are provided in corresponding locations in the facing edge of the other shell. Upon juxtaposition of the two shells the dowels are received into the corresponding recesses thus holding the two parts together.

The camera unit housing shells or half-housing may each comprise a side wall, a base portion and sleeve portion.

The sleeve portions are adapted such that when the shells are joined there is provided an entry for the cable into the camera housing. The sleeve portions may be so formed as to form a cable restraint when the halves are joined, and thus prevent the cable end from being inadvertantly disconnected from the electronic circuitry within the camera head.

In order to seal the cable entry, a high temperature resistant, fluid-tight sealing compound may be applied between the cable and the sleeve portions.

The housing is also provided with means to permit visual image to read the image sensing means. Normally an aperture is formed in the housing adjacent the image sensing device. In a preferred embodiment a window is formed in the housing body to permit the entry of light and exclude other materials thereby maintaining the integrity of the environment within the housing.

A recess or bore may be formed in the housing in the bottom of which is located, in a sealed engagement, a lens or other light transmitting structure. The sides of the bore are preferably provided with an internal screw thread which co-operates with an external thread mounted on a suitable endoscope. Thus when the endoscope and camera head are coupled optical images may be transmitted directly from the endoscope output to the image sensor via the window. Other optical processing means such as filters for example infra-red filter are lenses for example for focusing and/or power change may be included in the light path between the endoscope and the image sensing means eg. CCD.

The container for holding and maintaining the camera head and cable in a sterilised state may be of air impervious material which is stable whilst the unit is being stored. The material employed for the container should be stable to the sterilising medium employed for example ethylene oxide. Suitable materials may include plastics such as polyethylene or PVC or metal foils such as aluminium foil.

The present invention also provides a video system for monitoring endoscopic surgical procedures comprising a video monitor, camera control unit, light source, and endoscopic instrument for imaging surgical activity and a disposable camera unit as described hereinj.

The present invention will be further illustrated by reference to the accompanying drawings in which:

Figure 1 is a block diagram of equipment utilised in endoscopic surgical practice and including the pre-packaged disposable imaging device of the present invention;

Figure 2 is a view in elevation of a camera and connector cord unit construced in accordance with the present invention.

Figure 3 is a block diagram of the electronic components of the disposable unit shown in Figure 2;

Figure 4 is a sectional illustration showing individual conductor array of the connector cable;

Figure 5 is a view in vertical section through the camera head of the present invention;

Figure 6 is a front view in elevation of the camera head of Figure 2;

Figure 7 is a sectional showing through the camera housing illustrating the mating engagement of the housing frame;

Figure 8 is an enlarged view illustrating the base portion and strain relief sleeve of one-half of a camera housing;

Figure 9 is a section taken through the strain relief sleeve at lines 9-9 of Figure 8; and

Figure 10 is an illustration of an adaptor that may be utilised in connecting the connector cable to the camera control unit.

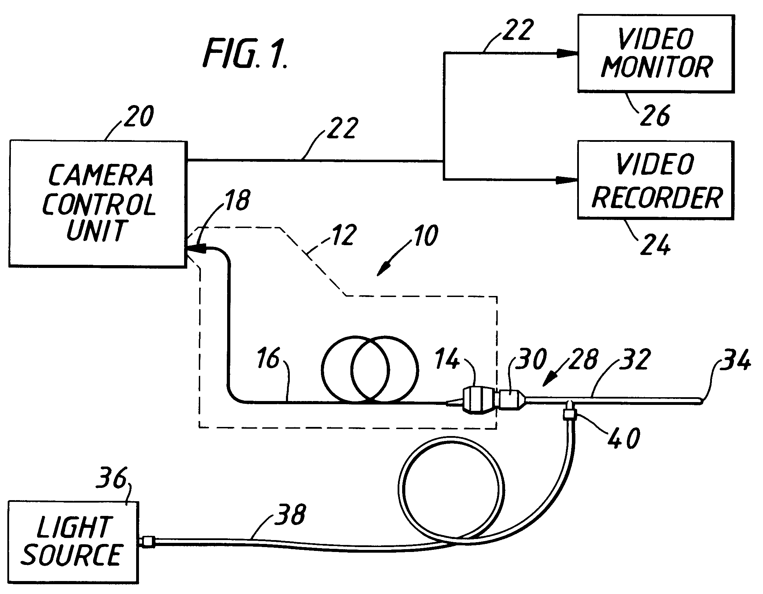

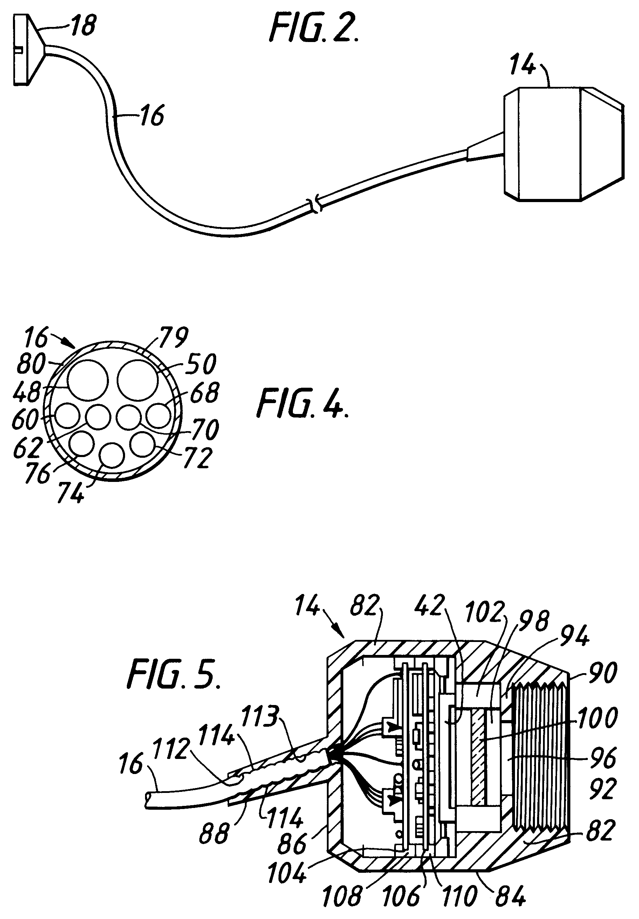

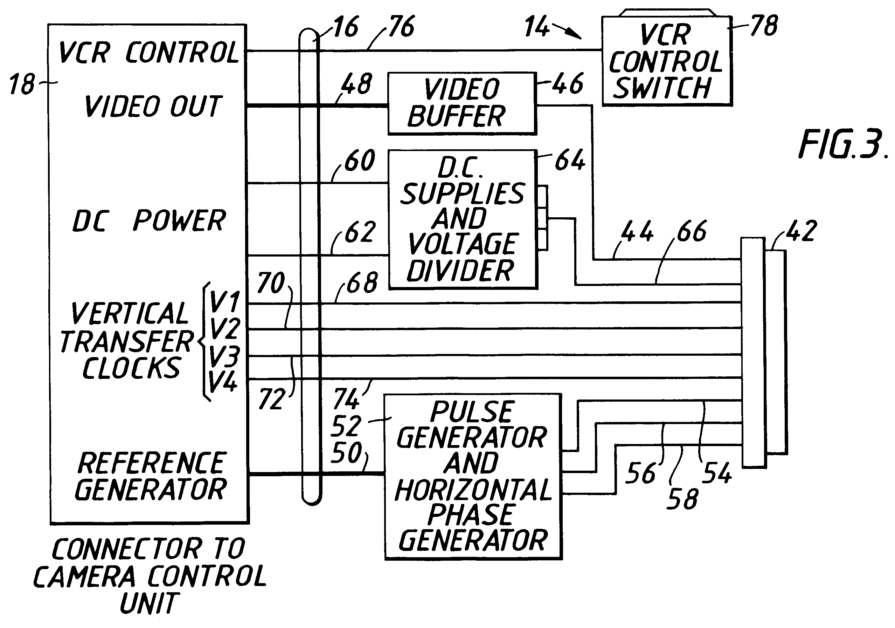

Figure 1 illustrates equipment utilised in the performance of endoscopic surgery, including the sterile connector/camera unit 10 as contained in a sterile pack, illustrated generally by dash line 12 and as shown in Figure 2. The unit 10 consists of a camera head 14, connected by means of cable 16 and plug connector 18 to a camera control unit 20. The camera control unit 20 serves as primary power-er and control source providing operational voltage supply to camera head 14 while also providing video and scanning output voltages via line 22 for input to a respective video recorder 24 and video monitor 26.

Light input to camera head 14 is provided by means of a scope instrument such as an arthroscope 28. Arthroscope 28 is directly connected to camera head 14 via a scope head 30 which may be affixed by a threaded connection into the front of camera head 14. The arthroscope 28 consists primarily of an elongated tube 32 that carries an objective lens and a plurality of optical fibres terminating at a viewing tip 34. Thus, any view apparent at tip 34 is conducted by fibre optics for presentation to the imaging surface within camera head 14, as will be further described. Intermediate along tubing 32 is a birefringent interface that allows introduction of an external light source through the viewing tip 34. Thus, a suitable light source 36 provides output through a plurality of fibres of an optical cable 38 which is plugged in at a junction connector 40 wherein reflection from the birefringent reflector in tubing 32 delivers light on the subject through the viewing tip 34.

The camera control unit 20 and light source 36 are standard equipment and suitable units are commercially available from Smith & Nephew Dyonics Inc, Andover, Massachusetts, USA. The arthroscope 28 and light source and conductor accessories are also available from the above company. Ancillary equipment such as video monitor 26, video recorder 24 and other support devices may be acquired from any of various commercial suppliers, eg. Panasonic Corporation.

Figure 3 illustrates the connector cable 16 and electronics of camera head 14. An image sensor 42 within camera head 14 provides optical pickup and output of video electrical data. The image sensor 42 may be a suitable charge-coupled device, for example an Amperex Type KX-1020. Video signal output via a lead 44 is applied through a video buffer 46 for output via a coaxial cable 48 within the cable connector 16. Scan frequency output from the camera control unit 20 (Figure 1) provides a reference generator input on a coaxial cable 50 of cable 16 to a stage 52 in camera head 14 which functions to generate frame and horizontal phase pulse output. The pulse generator 52 provides output via leads 54, 56 and 58 to the image sensor 42. The video buffer 46, pulse generator circuitry 52 and D-C control circuitry may be constructed of selected types of transistor logic intregrated circuites of known type.

D-C power from camera control unit 20 is supplied via leads 60 and 62 to a D-C supply and voltage divider stage 64 in camera head 14. Voltage divider of supply stage 64 then provides output via lead 66 of a D-C bias voltage to image sensor 42. Vertical transfer clock pulses from camera control unit 20 are successively output on respective leads 68, 70, 72 and 74 for direct connection to the image sensor 42. Finally, a lead 76 provides switch indication from a recorder control switch 78 for control application through a camera control unit 20. The switch 78 may be a suitable form of push button switch, eg. a membrane switch that is actuable through the sealed housing of camera head 14. As shown in Figure 4, the cable 16 includes 2 coaxial cables 48 and 50 while the remaining seven leads 60, 62, 68, 70, 72, 74 and 76 are 30-gauge wires. the cable is contained in a braided shield 79 and sheathing 80 of a suitable resilient cable material that is susceptible of sterilisation treatment, eg. a polyethylene sheathing.

Referring to Figure 5, the camera 14 is preferably formed in unitary or suitably continuous manner, thus aiding in keeping sterilisation, and it is designed to be compact and readily sealed against fluid incursions. Thus, the camera housing 82 may be formed of a suitable plastic, eg. a styrene polymer such as ABS. The housing 82 is preferably formed with a side wall 84, a base portion 86 and a strain relief sleeve portion 88 extending generally axially therefrom. The forward part of side wall 84 terminates at a rim 90 defining a threaded bore 92 which is adapted to receive threaded connection of the standard size arthroscope or other endoscopic instrument. There are a limited few such standard sizes and selected adaptors enable interchangeability. Housing 82 is further formed with an annular shoulder 94 which defines an aperture 96 that allows light transmission through a filter chamber 98 to the image sensor 42. A linear infra-red filter 100 may be disposed in filter chamber 98 and sealingly retained therein by means of a resilient mounting ring 102. An objective lens (not shown) projecting image ligh through filter 100 onto sensor 42 is retained in the associated scopic instrument.

Electronic components are arrayed on miniature circuit boards 104 and 106 as they are retained by respective moulded insert locators 108 and 110. The individual wires and coaxial cable connections from cable 16 are connected to the respective circuit boards 104, 106 as cable 16 is clamped with the strain relief sleeve 88 to effect a tight seal. The sleeve 88, to be further described below, indicates a plurality of spaced ridges 113 about its inner circumfery and, on assembly, a suitable sealant 112 is applied in the grooves 114 between respective ridges 113 and tightly secured in surround of cable 16 to effect a fluid-tight seal suitable for withstanding leakage during normal sterilisation procedures.

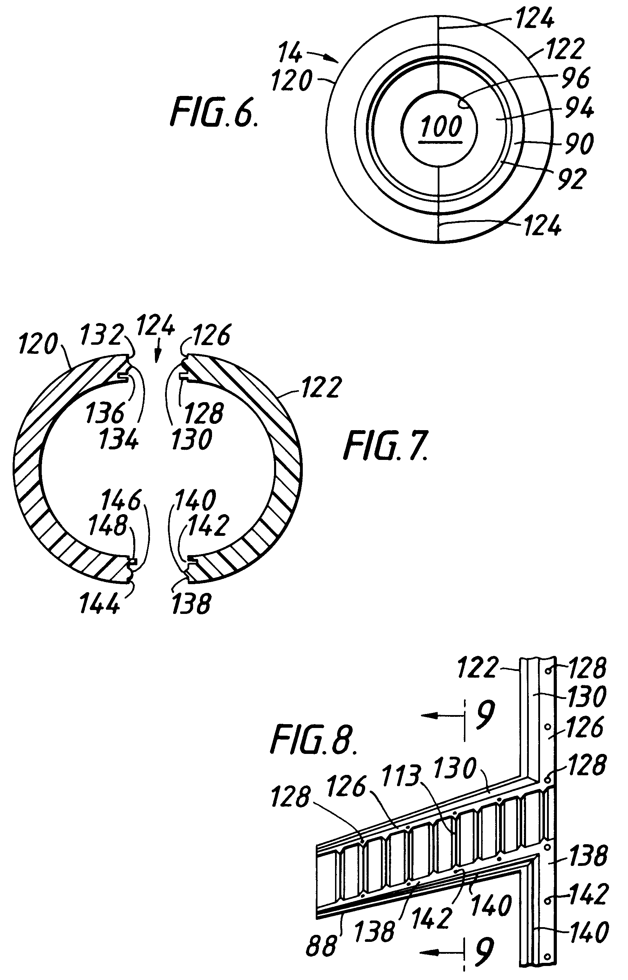

Figure 6 illustrates a front view of the camera head 14 as it is unitarily moulded into two-halves structure from a suitable plastic selected for resistance in the particular environment to be encountered. Thus, the camera head consists of a first housing half 120 and a second housing half 122 joined along a seam 124. The mouldings form annulus 94 defining aperture 96 through which light reflected from the subject source is presented through filter 100 to the image sensor 42 (Figure 5). An objective lens (not shown) projecting subjective light through filter 100 is an integral component of the arthroscope or other endoscope used in conjunction with the camera head. The scope instrument is secured in outer rim 90 within the threaded bore 92.

As shown in Figure 7, the two housing halves 120 and 122 are joined along seam 124 by facing which include both a dowel/pin arrangement and a ring/point sealing engagement. Thus, the upper joinder face 126 of half 122 includes a series of dowel/pins 128 as well as an O-ring formation 130 extending therealong. The upper face 132 of half 120 includes a point or ridge formation 134 and series of dowel holes 136 for mating engagement when the face halves are joined together in sealed formation to form seam 124. The seam 124 along the lower side of camera head 14 is similar but opposite as the dowel and ring/point surfaces are reversed. That is, bottom face 138 of half 122 includes a ridge line 140 and series of dowel holes 142, and lower face 144 of half 120 includes a semi-circular ring line 146 and series of dowel/pins 148.

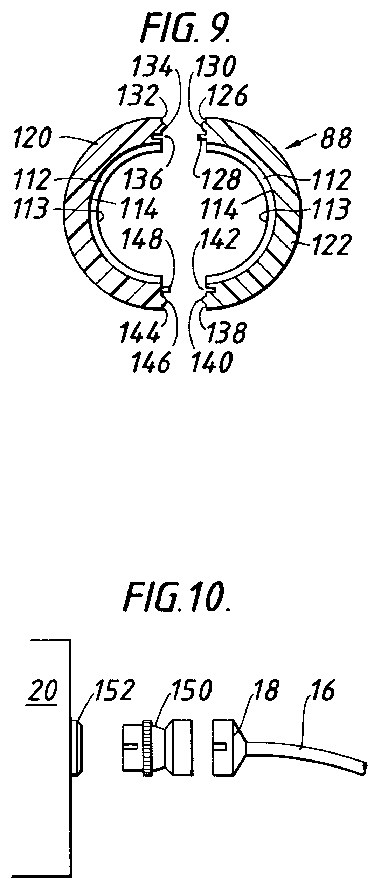

This structure is shown to better advantage in an enlarged rear section of the housing half 122 as shown in Figure 8. This view illustrates the manner in which the housing half defines part of the camera housing 14 and that extends rearwardly to form one part of the strain relief sleeve 88 to further aid in bring about a sterile, fluid-tight seal as between the connector cable 16 and the components within the camera housing. Figure 9 illustrates the ring/point and dowel/pin arrangement along sleeve 88. In assembly, a suitable sealant such as silicon latex compound is applied within the grooves 114 of sleeve 88 whereupon the halves 120 and 122 are then joined together with sleeve 88 (ridges 113) tightly seizing the cable 16 therein. A selected bonding agent is applied to the respective upper joinder faces 126, 132 and lower joinder faces 138, 144 to secure the camera head 14 with seam 124 consilidated on both top and bottom.

Figure 10 illustrates an alternative that may be required in connecting the cable 16 and plug connector 18 into the particular camera control unit 20. In some cases, a suitable adaptor 150 may be utilised in effecting the connection through connector receptacle 152 to the camera control unit 20. There are several standard sizes of connectors which the various control unit manufactureres utilise, and utilisation of adaptor connectors 150 will provide total interchangeability.

In operation, any problems as to sterilisation maintenance, operability and the like are overcome initially when, upon assembly, the unit 10 is tested, completely sterilised and encapulated in a suitable sterile pack. Sterilisation may by by immersion in ethylene oxide (ETO) or other recognised and acceptable practice. In standard procedure, the unit 10 in sterile form would be wrapped in sterile cotton cloth and then enclosed in a suitable transparent or translucent paper or plastic bag capable of maintaining tight seal and sterility over a long period of time. Such disposable camera units 10 are then available for shipping and shelf storage at operating facilities in whatever volume might be foreseen. The attendant electronics such as camera control unit 20 and the recording and monitoring hardware is of permanent installation and maintained in readiness in the endoscopic surgical operating room. In like manner, the light source 36 and fibre optics cable 38 with attendant control structure and arthroscope 28 and/or other scope instruments is kept in readiness.

When a surgical operation is to be performed, an attending nurse need only select from storage a sterile pack 12 containing the camera unit 10 which can then be unwrapped and connected between the camera control unit 20 and the operating position by attachment of camera 14 to the arthroscope 28. Cable 16 may be of about 4 metres (about 12 feet) in length to offer ample slack for movement about an operating area without interference. Simiarly, the fibre optics cable 38 is of sufficient length to enable free access and movement. The arthroscopic or other endoscopic surgery is then performed through completion whereupon the patient is removed to a post-operative area and the camera unit 10, ie. camera head 14 and cable 16, may be disconnected and disposed of as far as further surgical use is concerned.

The use of the camera unit and video systems in accordance with the invention enables greater assurance of surgical sterility while eventually serving to reduce the cost overall of performing arthroscopic surgery. The concept of a disposable camera is attractive to the attending physician and nurse staff for not only a decrease in the cost of operations, but also the provision of greater assurance as to sterility in and around the operating area. Thus, sterilisation of equipment at a central specialist agency enables elimination of dangerous and even poisonous sterilisation materials from the operating area and related laboratories. In addition, the disposable usage of camera units provides an operative tool that is assured to be of maximum acuity and resolution without requiring additional tuning or peaking operations.

The practice also provides greater assurance against failure of individual camera components, electrical connections and the like. One of the chief sources of failure with the prior surgical camera units is the multi-conductor connector cable as repeated sterilisation procedures can cause opening of one or more of the diminutive conductors encases therein. When this occurs, the entire operating procedure must be stopped or slowed as new camera equipment (if available) is interconnected and any additional sterilisation procedures are effected. The present method obviates such emergency measures as a new, sterile connector/camera unit is unveiled for use with each operation.

## Claims

1. A video camera unit for use in surgical procedures comprising:

a sterilised imaging sensing means including electronic control circuitry;

a sterilised connector cable having multiple conductors and being of pre-selected length having a plug connector on one end and having the other end connected to said control circuitry;

a sterilised housing means retaining said imaging device and control circuitry in fluid-tight, sealed connection to said connector cable; and

isolation means maintaining said imaging means, connector cable and housing means sterile.

2.  A unit according to claim 1 wherein said imaging sensing means is a solid-state, charge-coupled device.

3.  A unit according to claim 1 or claim 2 wherein said housing means comprises a hollow body of revolution bisected for assembly purposes and sealably closed to provide a fluid-tight container.

4.  A unit according to any one of the preceding claims wherein said housing means comprises a generally cylindrical housing bisected to define first and second half-housings each having a side wall, base portion and sleeve portion, said half housings being adapted to be juxtaposed and provide mating engagement.

5.  A unit according to claim 4 wherein each half is provided with a mating face which is adapted to mate with the other face and each face includes sealing means thereby to permit both halves to be mated in a sealed engagement.

6.  A unit according to claim 5 wherein said sealing means comprises:

a ridge of resilient material extending around one of said first or second mating faces; and

a ridge of non-resilient material extending around the other of said faces.

7.  A unit according to either claim 5 or claim 6 wherein one of said faces is provided with a pluarity of dowelling pins extending around said face and the other face is provided with cooperating recesses which are adapted to receive the dowelling pins when the faces are mated together.

8.  A unit according to any one of claims 4 to 7 wherein a high temperature resistant, fluid-tight sealing compound applied between the connector cable and said first and second half-housing sleeve portions.

9.  A unit according to any one of the preceding claims wherein said isolation means comprises:

a container of air impervious material for maintaining sterile condition of content during shelf life.

10.  A unit according to any one of the preceding claims wherein:

said housing means includes a light entry bore and means for securing a scope instrument in alignment therewith.

11.  A video system for monitoring endoscopic surgical procedures comprising a video monitor, camera control unit, light source an endoscope instrument for imaging surgical activity, and a disposable camera unit as claimed in any one of the preceding claims.

FIG. 1.

EP 0 496 142 A1

FIG. 2.

18

14

16

16
79
80
50
48
68
60
70
62
72
76
74

FIG. 4.

FIG. 5.

14  82  42  102  98  94
90
113
114  100
112  114
16  96
92
88  114  82
86
104
108  106  110  84

FIG.3.

FIG.6.

FIG.7.

FIG.8.

# FIG. 9.

# FIG.10.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| E | US-A-5010876 (G.D. HENLEY ET AL.) <br> * the whole document * <br> --- | 1-11 | A61B1/04 <br> A61B19/08 <br> H04N5/225 |
| X | US-A-4756304 (R.S. WATANABE) <br> * column 2, lines 3 - 22 * <br> * column 3, lines 4 - 52; figures * <br> --- | 1-3, 9, 10 | |
| X | US-A-4878485 (E.L. ADAIR) <br> * column 3, line 64 - column 5, line 36 * | 1, 2, 10, 11 | |
| Y | * figures * <br> --- | 3-5 | |
| Y | PATENT ABSTRACTS OF JAPAN <br> vol. 8, no. 155 (E-256)(1592) 19 July 1984, <br> & JP-A-59 057580 (KONISHIROKU SHASHIN K.K.K.) 03 April 1984, <br> * the whole document * | 3-5 | |
| A | | 7 | |
| A | US-A-4600940 (E. SLUYTER) <br> * column 4, line 6 - column 6, line 7; figures * <br> ----- | 1-4, 10, 11 | TECHNICAL FIELDS SEARCHED (Int. Cl.5 ) <br><br> A61B <br> H04N |

The present search report has been drawn up for all claims

| Place of search | Date of completion of the search | Examiner |
|---|---|---|
| THE HAGUE | 13 SEPTEMBER 1991 | RIEB K.D. |

EPO FORM 1503 03.82 (P0401)